# EUROPEAN PATENT APPLICATION

(11) **EP 1 038 835 A1**
(43) Date of publication of application: **27.09.2000**
(21) Application number: 00302257.1
(22) Date of filing: 20.03.2000
(51) Int. Cl.: C02F 1/32, B01J 19/12, B01J 19/24, A61L 2/10

(54) **Effluent treatment plant**

(30) Priority: 20.03.1999 GB 9906372
(71) Applicant: UV Systems PLC., Sudbury, Suffolk CO10 2QL (GB)
(72) Inventor: Mathieson, Mark, Suffolk C010 7LB. (GB); Mathieson, Colin, Suffolk C010 9DA. (GB)
(74) Representative: Gillam, Francis Cyril

(57) **Abstract**

A liquid treatment plant has a tank 10 within which is mounted a tubular treatment chamber 11 having its axis generally vertical and arranged to allow liquid to flow from the tank 10 into the lower end 18 of the chamber and upwardly therethrough. The upper end of the chamber 11 is arranged as a weir over which liquid may flow into an outlet duct 26. A hollow core 27 is disposed co-axially within the chamber 11 and has its upper end also arranged as a weir so that liquid may overflow into the core. The lower end of the core 27 is connected to the outlet duct 26 and UV disinfection treatment lamps 22 are disposed within the annular flow passage between the chamber wall and the core.

## Description

This invention primarily relates to an effluent disinfection plant, and also to an effluent treatment plant suitable for treating effluent other than by disinfection, but in certain aspects the invention further relates to the treatment of liquids other than effluent.

The treatment of effluent, and primarily sewage, by means of ultraviolet (hereinafter UV) radiation is well known and widely used. The normal procedure is for the sewage to be fed along a generally elongate horizontal channel in which is disposed a plurality of banks of UV discharge tubes arranged side by side across the channel, each bank having several tubes disposed horizontally, one above the other. In a large installation, there may be several sets of banks, disposed along the length of the channel. In this way, there may be a reasonable assurance that all of the effluent flowing along the channel is subjected to UV radiation, thereby disinfecting the effluent.

The procedure as described above is in general suitable only for large scale sewage works, in view of the length of channel required to obtain sufficient disinfection of the sewage, and also the requirement to have an open channel. The channel usually is covered by suitable lids, but then access for maintenance, replacement of the UV tubes (which have a limited life) and general servicing is more difficult and time-consuming, in view of the large number of lids which must be removed and later replaced.

It is a principal aim of one embodiment of the present invention to provide an effluent disinfection plant suitable for construction on a relatively small scale, such as to service the sewage discharge from a small village or just a group of houses.

Accordingly, one aspect of the present invention provides a liquid effluent disinfection plant which comprises a tank into which the effluent is introduced, a disinfection chamber mounted within the tank and arranged so that liquid flowing into the tank flows through the chamber, at least one elongate UV source mounted within the chamber to effect disinfection of liquid flowing through the chamber, and an outlet duct leading out of the tank from the chamber. Such a plant is characterised in that the chamber is tubular and is mounted with its axis disposed substantially vertically, in that the chamber permits the flow of liquid from the tank into the lower end of the chamber, and in that there is a gallery surrounding the upper end of the chamber and into which liquid rising up the chamber overflows.

It will be appreciated that the disinfection plant as described above may be installed in a relatively small space, since the tubular disinfection chamber is disposed generally vertically and the effluent flows upwardly through that chamber whilst being subjected to the disinfection process. The tank within which the process is performed may be correspondingly small and closed except for an access opening for servicing the UV source and a single lid may be provided for that opening. Removal of a single lid does not cause any great difficulty nor restrict maintenance, unlike the situation with an elongate horizontal channel where a large number of lids must be provided, and removed for servicing.

It has been determined that better disinfection by UV may be obtained by providing an internal core within the chamber, so as to define an annular flow passage for effluent rising upwardly through the chamber. Preferably, both the chamber and the core have substantially circular cross-sections, the core being mounted co-axially with the chamber and the UV source(s) being arranged within the annular space. In this way, enhanced UV disinfection of the effluent may be obtained, by avoiding slow-moving areas of effluent, within the chamber.

The avoidance of slow-moving areas may further be improved by extracting disinfected effluent both from the central region of the chamber and around the upper edge of the chamber. Advantageously therefore, the core is hollow and defines at its upper end a weir over which liquid rising up the chamber overflows, a pipe then being arranged to connect the lower end of the core to the outlet duct from the tank. In this way, liquid flowing over the weir and into the core is fed to the outlet duct, for mixing with treated effluent overflowing the upper end of the chamber, into the gallery and thence into the outlet duct.

The UV source mounted within the annular passage preferably comprises a plurality of UV discharge lamps each contained within a respective transparent sleeve and extending generally vertically into the passage. The upper end of each sleeve should be supported above the normal liquid level in the tank and through which upper end connections may be made to the contained UV discharge tube. The lower end of each sleeve should be sealed and preferably each tube is of the so-called single-ended kind.

There is entrained in liquid sewage a significant quantity of particulate matter and there is a tendency for this to settle on static surfaces such as a sleeve of a UV discharge tube. Preferably therefore a cleaning arrangement is provided for each UV source within the annular tube. For example, such an arrangement may comprise a mechanically-driven wiper for the sleeves surrounding the UV tubes, to remove the particulate matter. In addition, an ultrasonic generator may be arranged to impart sonic vibrations to the sleeves surrounding the UV tubes, thereby to dislodge particulate matter therefrom.

Tests on an effluent disinfection plant as described above have shown that the arrangement of an annular treatment passage has significant advantages for ensuring all of the liquid flowing upwardly through the chamber is subjected to the treatment process. For example, a catalytic or other treatment process may be performed on effluent flowing upwardly through the annular tube. It has thus been recognised that the plant can be used for the treatment of liquids other than effluent, as such, as well as for effluent treatments other than by UV disinfection.

Accordingly, a second aspect of the present invention provides a liquid treatment plant which comprises a tank into which the liquid is introduced, a treatment chamber mounted within the tank and arranged so that liquid flowing into the tank flows through the chamber, liquid treatment means disposed within the chamber and an outlet duct leading out of the tank from the chamber. Such a plant is characterised in that the chamber is tubular and is mounted with its axis disposed substantially vertically, in that the chamber permits the flow of liquid from the tank into the lower end of the chamber, in that there is a gallery surrounding the upper end of the chamber and into which liquid rising up the chamber overflows, and in that there is a hollow internal core disposed within the chamber so as to define a generally annular flow passage for liquid rising upwardly through the chamber and into which core the liquid may overflow, a pipe connecting the lower end of the core to the outlet duct whereby liquid flowing downwardly through the core is fed to the outlet duct to mix with liquid in the duct after overflowing the chamber into the gallery.

Such a treatment plant may have a similar arrangement of chamber and core to that of the disinfection plant described previously. A treatment plant and a disinfection plant both of this invention may employ baffles arranged preferably in the vicinity of the lower entrance to the passage or possibly even within the annular passage, to control the flow of liquid upwardly through the chamber. Preferably, such baffles are disposed to impart to the upward liquid flow a rotational component, about the axis of the chamber. In this way, stagnant areas of liquid may be minimised and the proper treatment of all of the liquid assured.

It will be appreciated that two or more effluent disinfection plants of this invention, or two or more liquid treatment plants also of this invention, may be arranged serially, with the outlet duct from the first plant being connected to the inlet to a subsequent plant. In the alternative, a plurality of such treatment plants may be arranged in parallel. For example, several such plants could be disposed within a relatively large chamber to which the liquid is introduced and the outlet ducts from all of the plants leading to a common discharge from the large chamber.

By way of example only, one specific embodiment of UV disinfection plant constructed and arranged in accordance with this invention will now be described in detail, reference being made to the accompanying drawings, in which:-
Figure 1 is a diagrammatic view of the embodiment of the disinfection plant;
Figure 2 is a detailed view on an enlarged scale of the upper end of the tubular disinfection chamber used in the plant of Figure 1;
Figures 3A and 3B are assembly drawings of the upper and lower ends respectively of the disinfection chamber;
Figure 4 is a plan view on the disinfection chamber;
Figures 5A and 5B diagrammatically illustrate a wiper arrangement;
Figure 6 diagrammatically illustrates a cascade of three disinfection plants, arranged serially; and
Figure 7 diagrammatically illustrates a treatment plant having a number of treatment chambers arranged in parallel.

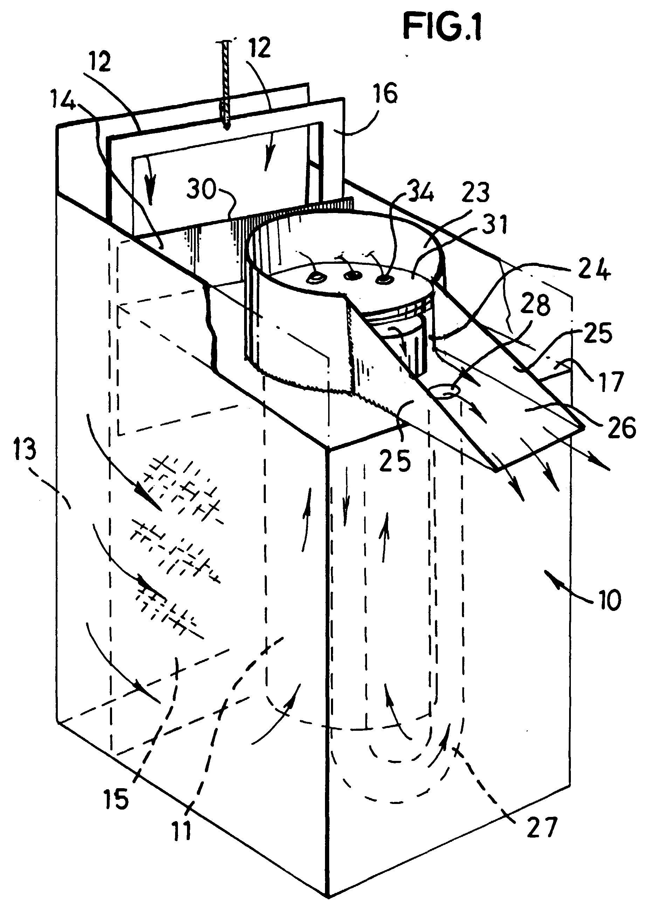

Referring initially to Figure 1, there is shown a tank 10 within which is mounted a disinfection chamber 11, for effluent introduced into the tank as shown at 12, into an inlet region 13 defined by a separating wall 14. A removable strainer 15 is disposed over the opening between the lower edge of separating wall 14 and the remainder of the tank 10, a lifting frame 16 being provided to permit removal of the strainer 15, for cleaning. Introduced effluent must thus pass through the strainer 15, to enter the main part of the tank 10.

A support plate 17 is mounted above the main part of the tank and has a central opening within which is mounted the disinfection chamber 11. The lower end 18 of that chamber is closed by a bottom plate 19 above which are provided four openings 20 equi-spaced around the chamber. Each opening 20 has an external cowl 21 arranged to induce a swirling motion to liquid entering the chamber through the respective opening 20, to avoid stagnant or slow-moving regions of liquid within the chamber 11.

A generally C-shaped wall 23 upstands from the support plate 17 and surrounds the upper end of the chamber 11 which projects above that support plate 17. An annular gallery 24 is thus formed between wall 23 and the part of the chamber 11 projecting above plate 17. Liquid rising up through the chamber 11 and overflowing the weir defined by the upper edge of the chamber 11 will flow into that gallery 24 and then exit through the open mouth of the C-shaped wall, between side plates 25 defining an outlet duct 26.

Mounted co-axially within the chamber 11 is a pipe 27, the upper end of the pipe being at substantially the same level as the upper end of the chamber 11. The pipe 27 is generally U-shaped and leaves the chamber 11 through the bottom plate 19, the other end of the pipe being sealed to an opening 28 in the outlet duct 26. In addition to the effluent overflowing the upper end of the chamber 11 directly into the gallery 24, the effluent may also overflow into the pipe 27 and from there flow downwardly out of the chamber and then back up the pipe to enter the duct 26, as shown by arrows 29.

The upper edge 30 of the separating wall 14 is below the upper edges of the side walls of the tank 10, so that should there be excess flow conditions or a blockage for example in the strainer 15, effluent may overflow that upper edge 30 on to the upper surface of the support plate 17, and from there reach the outlet duct 26 without passing upwardly through the chamber 11. However, during all normal operating conditions of the plant, no such overflowing of the separating wall 14 should occur.

Disinfection tubular UV discharge lamps 22 (not shown in Figure 1) are arranged within the annular space between the chamber 11 and the pipe 27, which lamps are energised whenever effluent flows upwardly through the chamber 11. Such lamps 22 irradiate the effluent with UV radiation and so perform a disinfecting action on that effluent. As an alternative to UV discharge lamps, the annular space could contain suitable material for performing some other effluent or other liquid treatment process, such as a catalyst to promote the performance of a digestion process on the effluent.

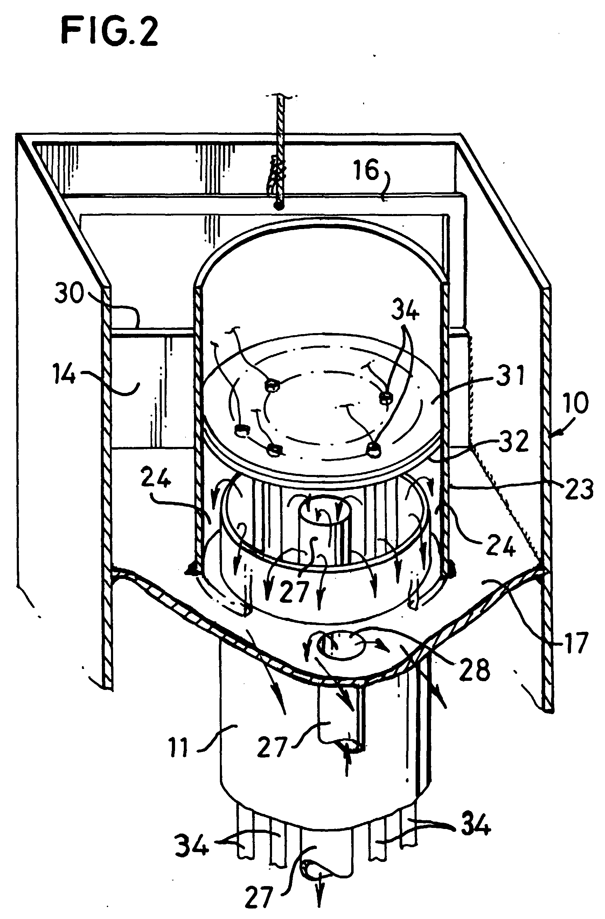

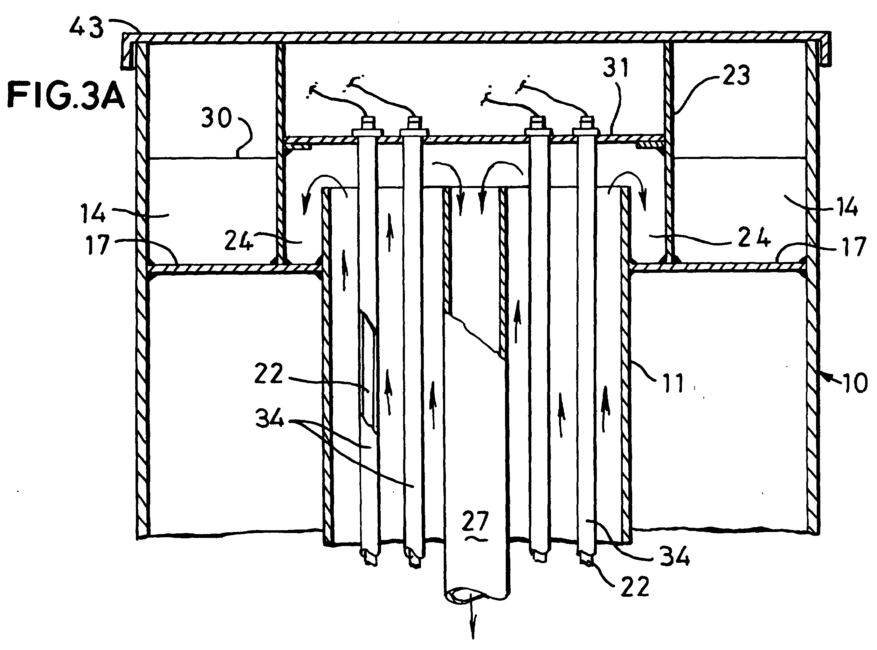

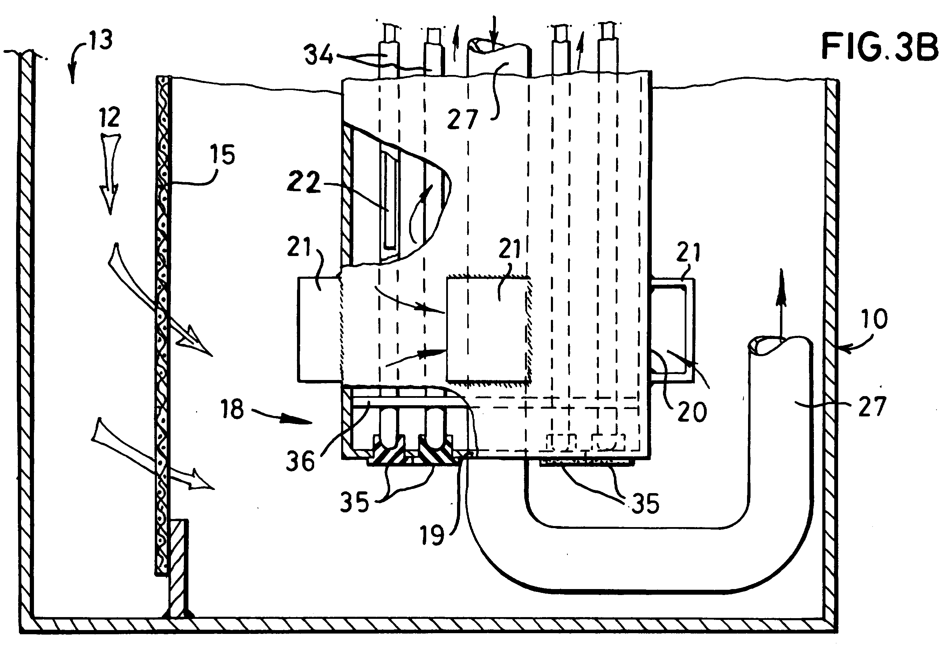

Figures 2, 3A and 3B are detail views on the upper and lower ends of the chamber 11. A mounting plate 31 is carried on a flange 32 projecting inwardly from the C-shaped wall 23, above the level of the upper edge of the chamber 11, and so above the normal liquid level in the tank. The mounting plate 31 has apertures 33 formed therein, a respective sleeve 34 being mounted in each aperture and containing a single-ended UV discharge lamp 22. Each sleeve 34 is of quartz and is sealed at its lower end; connections are made to the contained discharge lamp through the open upper end of the sleeve, exposed above the mounting plate 31. Though not shown in the Figures, the mounting plate 31 has downwardly depending supports for the upper end of pipe 27, to maintain that upper end at substantially the same level as chamber 11, and co-axial therewith.

At the lower end of the chamber 11, the bottom plate 19 has a respective bore aligned with each sleeve 34, rubber grommets 35 being provided in the bores to locate and hold the lower ends of the sleeves. The lower end of each discharge lamp 22 in its respective sleeve is above the plane of the liquid openings 20 at the lower end of chamber 11, so that all the UV radiation will treat incoming effluent.

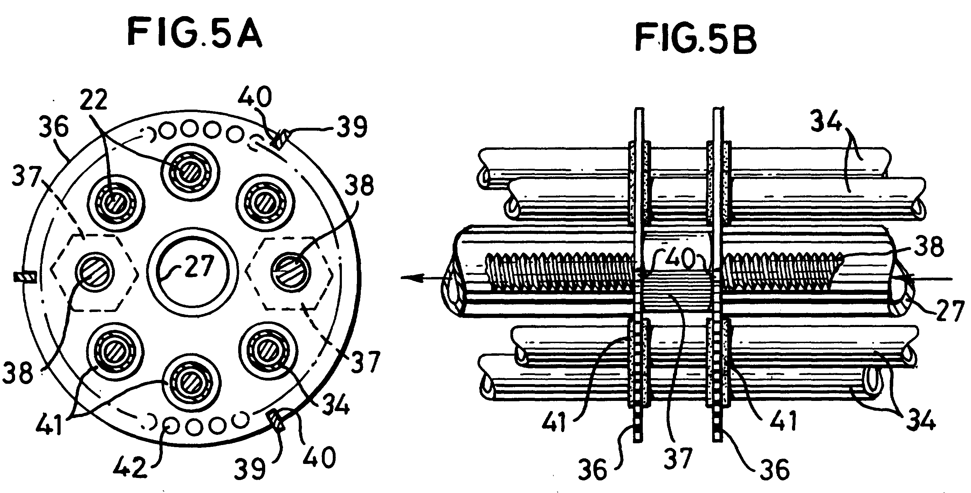

The sleeves 34 are provided with respective wiping arrangements, to remove particulate matter settling thereon. One possible form of such a wiping arrangement is shown in Figures 5A and 5B. This comprises a pair of plates 36 held in a spaced-apart parallel disposition by diametrically opposed nuts 37 attached to both plates 36 and threaded on to respective lead screws 38. Optional guide bars 39 pass through peripheral slots 40 in the plates 36 and serve to stop rotation of the wiper as the lead screws are turned. The arrangement of Figures 5A and 5B is simplified and intended for cleaning an array of six sleeves 34 each containing a UV lamp, each sleeve passing through further openings in the plates, which openings are filled with wiper elements 41 bearing on the surfaces of the sleeves. Typically, each wiper element may be in the form of a brush or soft bushing; preferably there is only one wiper element for each sleeve, with the wiper elements arranged alternately in the two parallel plates 36.

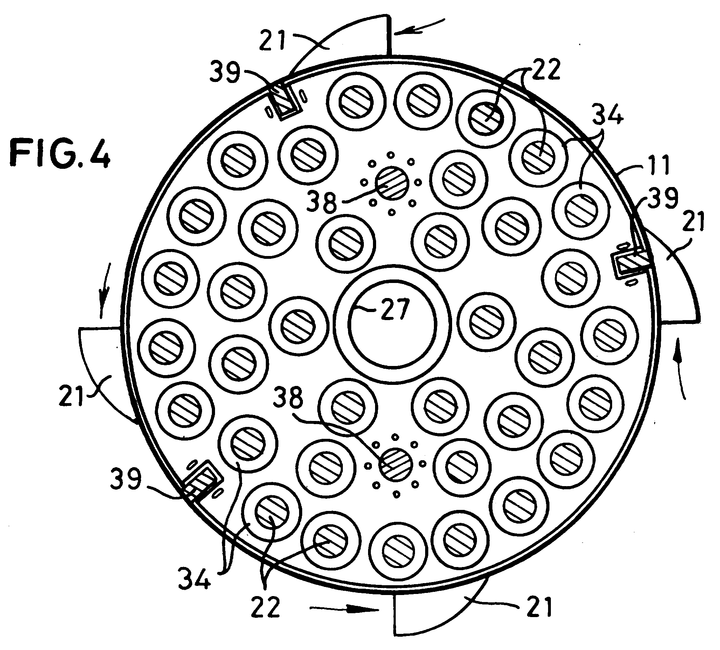

The wiping arrangement for the embodiment of Figures 1 and 2 is more complex, in view of the increased number of sleeves 34; in Figure 4, the wiping plate 36 is shown in plan view. A suitable motor drive arrangement (not shown) is provided on the mounting plate 31 for the two lead screws 38, so that simultaneous rotation of the two lead screws drives the wiping plates 36 along the length of the sleeves. In this way, the wiper elements scrape any particulate or sedimentary matter adhering to the sleeves. Further apertures 42 (Figure 5) may be formed in the plates 36 to minimise the liquid resistance to movement of the plates. When not in use, the wiping plates are parked at the bottom of the chamber 11, below the level of the openings 20, or the wiping plates could be parked immediately below the mounting plate 31, above the normal liquid level in the chamber.

As can be seen in Figure 3A, a lid 43 is provided for the plant and which is removable, to give access to the mounting plate 31, the lamps 22 and so on. For major servicing, the mounting plate 31 may be lifted out of tank 10, along with the chamber 11, the sleeves 34 containing the lamps 22, as well as the wiper arrangement.

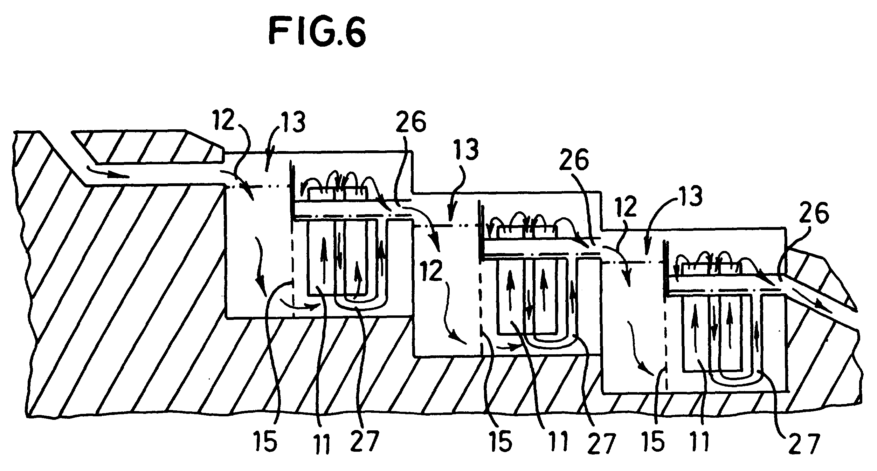

Figure 6 diagrammatically illustrates a series of three disinfection plants as described above, with the outlet duct 26 of one plant supplying the inlet region 13 of the next plant. The outlet duct 26 of the last plant in the series directs the fully disinfected effluent to a water-course, drain or the like.

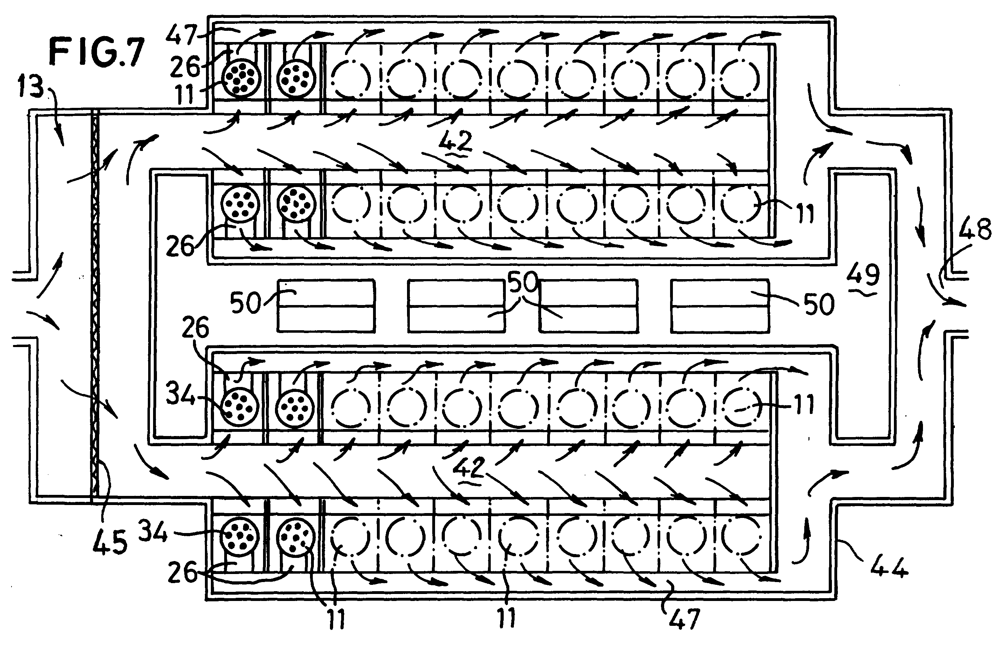

Figure 7 illustrates an alternative plant arrangement having multiple parallel disinfection chambers. A common tank 44 is provided for all of the chambers 11, which tank defines an inlet region 13 to which the effluent is introduced. A screen 45 is disposed between that inlet region and two inlet passageways 46 leading to the disinfection chambers 11. The arrangement of each chamber together with its internal and external components is substantially as described above and will not be described again here. The treated effluent leaves the outlet duct 26 of each chamber to enter discharge galleries 47 which in turn lead to an outlet passageway 48.

As can be seen from Figure 7, the chambers 11 are arranged in four banks, with a central clear region 49 between the two inner banks. Control cabinets 50 and other equipment may be provided within that clear region. It will be appreciated that by providing suitable gating arrangements (not shown) for each chamber 11, it is possible to take out of service one or more chambers for maintenance without significantly affecting the throughput of the overall plant.

In an alternative arrangement, the screen 45 could be omitted and each chamber 11 would then be provided with its own inlet region having an individual screen, as with the arrangement of Figures 1 and 2.

## Claims

1. A liquid effluent disinfection plant comprising a tank (10) into which the effluent is introduced, a disinfection chamber (11) mounted within the tank and arranged so that liquid flowing into the tank flows through the chamber, at least one elongate UV source (22,34) mounted within the chamber (11) to effect disinfection of liquid flowing through the chamber, and an outlet duct (26) leading out of the tank from the chamber, characterised in that the chamber (11) is tubular and is mounted with its axis disposed substantially vertically, in that the chamber (11) permits the flow of liquid from the tank (10) into the lower end of the chamber, and in that there is a gallery (24) surrounding the upper end of the chamber (11) and into which liquid rising up the chamber (11) overflows.

2. A liquid effluent disinfection plant as claimed in claim 1, wherein the chamber (11) has an internal core (27) so as to define in conjunction therewith a generally annular flow passage for liquid rising upwardly through the chamber (11).

3. A liquid effluent disinfection plant as claimed in claim 2, wherein both the tubular chamber (11) and the core (27) have substantially circular cross-sections, and the core is mounted with its axis substantially coincident with the axis of the chamber.

4. A liquid effluent disinfection plant as claimed in claim 2 or claim 3, wherein the core (27) is hollow and defines at its upper end a weir over which liquid rising up the chamber (11) overflows, the lower end of the core being connected to the outlet duct (26) whereby liquid flowing down the core (27) is fed to the outlet duct.

5. A liquid effluent disinfection plant as claimed in any of the preceding claims, wherein the UV source comprises at least one UV discharge lamp (22) contained within a transparent sleeve (34) and extending generally vertically within the chamber (11), in the liquid flow path.

6. A liquid effluent disinfection plant as claimed in any of the preceding claims, wherein cleaning means (41) are provided for the or each UV source mounted within the chamber (11), which cleaning means preferably comprises a mechanically-driven wiper (36,38,41) for the or each UV source, to remove particulate matter from the surface of the source exposed to the liquid flow.

7. A liquid treatment plant comprising a tank (10) into which the liquid is introduced, a treatment chamber (11) mounted within the tank and arranged so that liquid flowing into the tank flows through the chamber, liquid treatment means disposed within the chamber (11), and an outlet duct (26) leading out of the tank from the chamber, characterised in that the chamber (11) is tubular and is mounted with its axis disposed substantially vertically, in that the chamber (11) permits the flow of liquid from the tank (10) into the lower end of the chamber, in that there is a gallery (24) surrounding the upper end of the chamber (11) and into which liquid rising up the chamber (11) overflows, and in that there is a hollow internal core (27) disposed within the chamber (11) so as to define a generally annular flow passage for liquid rising upwardly through the chamber (11) and into which core the liquid may overflow, a pipe connecting the lower end of the core (27) to the outlet duct (26) whereby liquid flowing downwardly through the core is fed to the outlet duct to mix with liquid in the duct after overflowing the chamber into the gallery (24).

8. A liquid treatment plant as claimed in claim 7, wherein the treatment means is disposed in the generally annular passage and comprises at least one of a catalyst or other treatment material or means (22,34) for the treatment or disinfection of the liquid.

9. A liquid treatment plant as claimed in claim 7 or claim 8, wherein both the tubular chamber (11) and the core (27) have substantially circular cross-sections, and the core is mounted with its axis substantially coincident with the axis of the chamber.

10. A liquid treatment plant as claimed in any of claims 7 to 9, wherein the upper end of the core (27) is at substantially the same level as the upper end of the chamber (11).

11. A plant as claimed in any of the preceding claims, wherein baffles (21) are arranged to impart a swirling motion to liquid entering the chamber (11).

12. A plant as claimed in any of the preceding claims, wherein there is a weir (14,30) formed between an inlet to the tank (10) and the outlet duct (26), whereby during periods of excess flow rate or blockages in the chamber (11), liquid may flow direct from the inlet over the weir (14,30) to the outlet, by-passing treatment chamber (11).
